# EUROPÄISCHE PATENTSCHRIFT

(11) **EP 2 103 281 B1**
(45) Veröffentlichungstag und Bekanntmachung des Hinweises auf die Patenterteilung: **09.06.2010**
(21) Anmeldenummer: 09003942.1
(22) Anmeldetag: 19.03.2009
(51) Int. Cl.: A61F 2/18

(54) **Gehörknöchelchenprothese mit variablen Ankopplungsflächen**
Ossicular prosthetic with variable coupling surfaces
Prothèse de l'osselet de l'oreille dotée de surfaces d'accouplement variables

(30) Priorität: 20.03.2008 DE 102008015114
(43) Veröffentlichungstag der Anmeldung: 23.09.2009
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus

(56) Entgegenhaltungen:
- DE-A1- 1 926 587
- DE-B3-102007 013 708
- DE-U1- 29 802 776

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein im Wesentlichen plattenförmiges erstes Befestigungselement zur Anlage am Trommelfell oder an der Steigbügelfußplatte und an ihrem anderen Ende ein zweites Befestigungselement zur mechanischen Verbindung mit einem Glied oder Teilen eines Gliedes der Gehörknöchelchenkette oder mit dem Innenohr sowie ein die beiden Befestigungselemente Schall leitend miteinander verbindendes Verbindungselement umfasst, und wobei das plattenförmige erste Befestigungselement einen radial inneren, insbesondere um den Flächenschwerpunkt des plattenförmigen ersten Befestigungselements zentral angeordneten Ankoppelbereich zum mechanischen Ankoppeln des ersten Befestigungselements an das Verbindungselement sowie mehrere Stegelemente zur radialen Verbindung des radial inneren Ankoppelbereichs mit radial äußeren Abschnitten des ersten Befestigungselements aufweist, wobei die Stegelemente geometrisch derart gestaltet sind und ihr Material so gewählt ist, dass die Stegelemente vom plattenförmigen ersten Befestigungselement abgebrochen und die an ihnen anhängenden radial äußeren Abschnitte vom ersten Befestigungselement gelöst werden können.

Eine derartige Vorrichtung ist aus der DE 10 2007 013 708 B3, die als nächst liegenden Stand der Technik betrahtet wird, bekannt. Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung bzw. Signalübertragung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilungsphase. In dieser Zeit bilden sich Narben und Gewebestränge und diese verursachen unvorhersehbare Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität und Flexibilität aufweist, so dass sich die Kopfplatte nach der Operation selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells von Mensch zu Mensch variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen bekannt, die elastische Teile und/oder Gelenke aufweisen. Eine solche gelenkige Verbindung zwischen einem an der Steigbügelfußplatte montierbaren Befestigungselement und dem länglichen Schaft ist in der EP 1 181 907 B1 beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einher gehende akute oder chronische Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sog. Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet und meistens als länglicher Schaft ausgeführt ist. Eine solche in sich starre, aber gegenüber dem Verbindungselement verkippbare Kopfplatte ist u.a. beschrieben in der US 2004/0162614 A1, in dem Artikel M.W. YUNG, Ph.D., F.R.C.S., D.L.O., C. BREWIS, F.R.C.S., "A comparison of the user-friendliness of hydroxyapatite and titanium ossicular prostheses", The Journal of Laryngology & Otology, February 2002, Vol. 116, pp. 97-102, oder beispielsweise auch in der US 2006/0271190 A1.

Nachteilig bei diesen bekannten Gehörknöchelchenprothesen ist allerdings, dass durch die in sich starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Um eine hohe postoperative Flexibilität und Variabilität der Prothese zu erreichen, wobei gleichzeitig die Qualität der Schallleitung durch die Prothese erheblich erhöht werden soll, ohne dass es zu den oben geschilderten Komplikationen kommen kann, schlägt die eingangs zitierte DE 10 2007 013 708 B3 vor, dass die Stegelemente geometrisch so gestaltet sind, dass sie bei lokaler Medialbewegung des Trommelfells dieser Medialbewegung lokal folgen, jedoch die Bewegung nicht an entfernte Bereiche der Kopfplatte weitergeben. Durch diese flexible Gestaltung der Gehörknöchelchenprothese wird bei einer solchen kleineren Medialbewegung des Trommelfells eine starre Verkippung der gesamten Kopfplatte vermieden. Vielmehr verwindet sich die Kopfplatte lokal in sich selbst, wobei sie jedoch bei großflächigen, durch Schall verursachten Bewegungen des Trommelfells diese an das Verbindungselement weitergibt, so dass eine optimale Übertragung des Schalls bzw. des Schallsignals vom Trommelfell zum Mittelohrraum hin und weiter ans Innenohr gewährleistet wird.

Damit wird zwar gegenüber dem übrigen bekannten Stand der Technik eine ganz erhebliche Verbesserung erzielt. Leider bestehen jedoch noch weitere Probleme, die mit diesen Maßnahmen alleine nicht gelöst werden können:
Pathologie und Anatomie können im Rahmen einer Tympanoplastik im menschlichen Mittelohr ganz unterschiedliche, spezifisch vom individuellen Patienten abhängige Struktur-Rekonstruktionen notwendig machen. Je nach Ausmaß und Form von eventuell noch vorhandenen und vielleicht teilweise intakten Teilen der Mittelohranatomie, wie des Hammers (=Malleus), des Ambosses (=Incus), des Steigbügels (=Stapes) oder des Trommelfells, werden entsprechend viele, zum Teil ganz erheblich in Form und Größe voneinander abweichende Geometrien der einzusetzenden Mittelohrprothesen benötigt.
Da vor Beginn einer Mittelohr-Operation nur sehr schwer oder gar nicht vorhergesagt werden kann, wie sich die spätere Rekonstruktion des Trommelfells und der Gehörknöchelchenkette im Verlauf der Operation darstellen wird (wenn überhaupt, dann nur grob, aber praktisch niemals genau), müssen derzeit für jede aktuell durchzuführende Operation immer sehr viele Mittelohrprothesen mit unterschiedlichen Geometrien, Formen und Größen bereitgehalten werden, damit der Chirurg intraoperativ stets aus einer vorrätigen Vielfalt heraus die jeweils am besten passende Prothese auswählen kann, die es ihm ermöglicht, speziell auf die jeweilige Gegebenheit einzugehen. Ansonsten kann es dazu kommen, dass keine optimale Versorgung gewährleistet ist.

Hinzu kommt, dass die genannten intraoperativen Anpassungsprobleme der Gehörknöchelchenprothese nicht nur im Anlagebereich des ersten Befestigungselements an das Trommelfell auftreten können, sondern ebenso im Bereich eines gegebenenfalls erforderlichen, ebenfalls plattenförmigen zweiten Befestigungselements, das zur Anlage der Prothese auf der Steigbügelfußplatte dienen kann. Insbesondere für den zum Innenohr hin abschließenden Bereich einer Totalrekonstruktion weist eine Totalprothese zu diesem Zweck normalerweise einen Stempel mit einem Standard-Durchmesser von 0,8 mm auf. Oftmals wird von chirurgischer Seite aus der Wunsch geäußert, dass man gerne - je nach intraoperativer Situation - unterschiedliche Größenareale zur Verfügung hätte, die auf die Fußplatte des Steigbügels aufgesetzt werden können. Die Bereitstellung eines zusätzlichen, mit dem Stempel verbundenen oder verbindbaren Befestigungselements, welches hinsichtlich der Größe seiner Fläche in weiten Grenzen variabel wäre, würde diesem Wunsch der Fachwelt entgegen kommen.

Falls die Gehörknöchelchenprothese keine Totalprothese und damit das erste Befestigungselement nicht als Kopfplatte zur Anlage am Trommelfell ausgebildet ist, sondern als Klammer zur Befestigung der Prothese an einem Glied der Gehörknöchelchenkette, treten die beschriebenen Anpassungsprobleme eventuell auch ausschließlich am Innenohrseitigen Ende der Gehörknöchelchenprothese auf.

Ein weiteres Problem liegt darin, dass - Weltweit gesehen - äußerst unterschiedliche chirurgische Techniken angewendet werden, welche verschiedenartige Rekonstruktionen im Mittelohr postulieren. Diese erfordern entsprechend angepasste, voneinander in Form und Größe sehr unterschiedliche Mittelohrprothesen, welche wiederum während jeder Operation vorrätig gehalten werden müssen, um dem Chirurgen die Möglichkeit zur geben, die nach seiner Einschätzung jeweils für den aktuell vorgefundenen Fall optimale Methode anzuwenden.

Aufgabe der vorliegenden Erfindung ist es demgegenüber, eine gattungsgemäße Mittelohrprothese der eingangs beschriebenen Art mit möglichst einfachen technischen Mitteln unaufwändig und kostengünstig dahin gehend zu verbessern, dass die Anzahl der intraoperativ bereit zuhaltenden unterschiedlichen Prothesen ganz erheblich verringert, vorzugsweise auf eine einzige Standard-Prothese reduziert werden kann, ohne dabei die Möglichkeit zur optimalen Adaption der Prothese im konkreten Einzelfall zu verlieren.

Erfindungsgemäß wird diese Aufgabe auf ebenso überraschend einfache wie wirkungsvolle Art und Weise dadurch gelöst, dass die Stegelemente jeweils eine Sollbruchstelle mit minimaler Breite aufweisen. Damit bricht das Stegelement beim Abbrechen eines radial äußeren Abschnittes an einer definierten Stelle ab und der Außendurchmesser des ersten Befestigungselements wird in diesem Bereich verkleinert.

Das plattenförmige erste Befestigungselement der erfindungsgemäßen Mittelohrprothese, welches z.B. als Kopfplatte ausgestaltet sein kann, die im Rahmen einer Tympanoplastik gegen das Trommelfell gelegt wird, ist so aufgebaut, dass es in seiner Form und Fläche in sehr weiten Grenzen variabel ist. Die standardmäßig vorrätig gehaltene erfindungsgemäße Prothese lässt sich daher intraoperativ in allen Belangen ganz einfach, sehr variabel und äußerst zielgenau so umgestalten, wie es die vorgefundene patientenspezifische Situation jeweils gerade erfordert.

Einfache, ad hoc vornehmbare Veränderungen der erfindungsgemäßen Standard-Prothese, etwa hinsichtlich von Winkeln, Längen oder Flächengrößen, dienen einer erheblich verbesserten Adaption im konkreten Einzelfall. Somit bietet die erfindungsgemäße MittelohrProthese dem Operateur ein extrem hohes Maß an Variabilität und Flexibilität, ohne dass dazu die bisher übliche Bevorratung einer großen Vielzahl unterschiedlichster Prothesenformen, -größen und -geometrien erforderlich ist. Der Chirurg kann damit intraoperativ an der Prothese gezielte Veränderungen vornehmen, die es ihm ermöglichen, die Prothese speziell auf die jeweilige Gegebenheit einzustellen bzw. anzupassen.

Mit der erfindungsgemäß angewandten Technik ist es ganz leicht möglich, beispielsweise die Kopfplatte der Prothese durch einfaches Abbrechen von nicht benötigten radial äußeren Abschnitten in ihrer Größe so zu verändern, dass sie nur speziell gewollte Bereiche am Trommelfell berührt - in der Regel diejenigen, bei denen man genau weiß, dass sie für die akustische Übertragung eine wesentliche Rolle spielen.

Oftmals liegt der Fall vor, dass der Hammergriff am Trommelfell vorhanden ist - oder eben auch fehlt - je nach dem wie oft voroperiert, welche Operationsmethode angewendet und welche spezifische Maßnahme ergriffen wurde. Entsprechend kann bei einer erfindungsgemäß flächenvariabel gestalteten Kopfplatte durch den Operateur zielgenau und exakt richtig reagiert werden, also der für den Hammergriff verantwortliche Bereich abgenommen oder - je nach Bedarf - an der Kopfplatte belassen werden.

Ähnliches gilt auch für eine Ankopplung der Gehörknöchelchenprothese an die Steigbügelfußplatte, wo ebenfalls aufgrund der erfindungsgemäß flächenvariablen Gestaltung des entsprechenden plattenförmigen Befestigungselements eine bisher nicht gekannte intraoperative Flexibilität im Hinblick auf eine optimale Anpassung an die individuelle Patientensituation geboten werden kann.

Generell kann mittels der vorliegenden Erfindung durch einfaches Abbrechen von Teilen einer Trommelfell-Kopfplatte oder einer Steigbügelfußplatte eine speziellere Adaption an die individuell vorgefundene Anatomie des Patienten vorgenommen werden, die in dieser Form bislang höchstens durch die Verwendung von sehr aufwändig und teuer eigens von Hand angefertigten Mittelohrprothesen möglich war, wobei dann allerdings in den allermeisten Fällen eine weitere Operation erforderlich wurde, da die exakten Maße in der Regel erst durch eine erste Öffnung des Mittelohrraumes gefunden werden können.

Von der erfindungsgemäßen Grundidee kann in doppelter Weise profitiert werden, wenn beide Befestigungselemente plattenförmig mit abbrechbaren Stegelementen und daran anhängenden radial äußeren Abschnitten aufgebaut sind, wobei das erste Befestigungselement zur Anlage der Gehörknöchelchenprothese auf der Steigbügelfußplatte ausgebildet ist und das zweite Befestigungselement als ebene Kopfplatte zur mechanischen Verbindung mit dem Trommelfell dient.

Weiter ist es günstig, wenn das plattenförmige erste Befestigungselement (und gegebenenfalls auch das zweite) der erfindungsgemäßen Gehörknöchelchenprothese eine Dicke, insbesondere eine Blechdicke t zwischen 0,01mm und 0,5mm, vorzugsweise zwischen 0,1mm und 0,25mm, sowie einen minimalen Durchmesser D zwischen 1,5mm und 8mm, vorzugsweise zwischen 2mm und 5mm, und die Stegelemente eine maximale Breite b zwischen 0,01mm und 0,3mm, vorzugsweise zwischen 0,05mm und 0,2mm aufweisen.

Bei einer Klasse von vorteilhaften Ausführungsformen der erfindungsgemäßen Gehörknöchelchenprothese gehen - ausgehend vom zentralen Ankoppelbereich - Stegelemente sternförmig radial nach außen ab, wobei an diesen Stegelementen jeweils ein radial äußerer Abschnitt anhängt, und an jedem dieser radial äußeren Abschnitte über weitere Stegelemente jeweils weitere radial äußere Abschnitte anhängen.

Dadurch steht eine große Vielzahl von radial äußeren Abschnitten des ersten Befestigungselements zur Verfügung. Durch gezieltes Abbrechen einzelner oder ganzer Gruppen dieser Abschnitte kann nun fast jede beliebige Geometrie des Befestigungselements ohne weitere Hilfsmittel sehr einfach ad hoc hergestellt werden.

Bei einer alternativen Klasse von Ausführungsformen bilden die radial äußeren Abschnitte des plattenförmigen ersten Befestigungselements einen äußeren Ringbereich, der aufgrund seines hohen Flächenträgheitsmoments die Stabilität des Befestigungselements erheblich erhöht.

Eine Gruppe von Weiterbildungen dieser Ausführungsformen zeichnet sich dadurch aus, dass der radial äußere Ringbereich ununterbrochen in sich selbst geschlossen verläuft. Auf diese Weise kann wird die Stabilität des ersten Befestigungselements weiter erhöht. Falls dieses als Kopfplatte ausgebildet ist, kann außerdem bei post-operativen Retraktionen die Ausbildung von gegen das Trommelfell gerichteten, gefährlichen Spitzen vermieden werden.

Alternativ dazu ist bei einer anderen Gruppe von Weiterbildungen vorgesehen, dass der radial äußere Ringbereich mindestens eine, vorzugsweise mehrere Unterbrechungen aufweist. Hiermit lassen sich insbesondere asymmetrische Formgestaltungen leicht bewerkstelligen. Außerdem wird eine plastische Verformung des plattenförmigen ersten Befestigungselements durch Ziehen oder Stauchen des in Abschnitte geteilten Ringbereichs innerhalb der Plattenebene erleichtert, was weitere Möglichkeiten zur Formgebung eröffnet.

Der radial äußere Ringbereich kann bei Weiterbildungen der oben beschriebenen Ausführungsformen eine ovale oder kreisrunde Form aufweisen, was aus dem Stand der Technik an sich bekannt und einfach herstellbar ist. Dies wird in der Regel die Standard-Variante für die erfindungsgemäße Gehörknöchelchenprothese bilden.

Zur Erleichterung des operativen Einsetzens der erfindungsgemäßen Gehörknöchelchenprothese kann bei einer speziellen Variante der radial äußere Ringbereich eine einseitige Einbuchtung zur Aufnahme des Hammergriffs aufweisen.

Eine weitere Variante sieht vor, dass der radial äußere Ringbereich des plattenförmigen ersten Befestigungselements in der Plattenebene eine radial nach außen verlaufende Ausbuchtung aufweist, die in Strukturen der Gehörknöchelchenkette eingreifen kann.

Möglich ist aber auch eine Variante, bei der der radial äußere Ringbereich eine Schlangenlinienförmige Außenkontur aufweist, die sich in speziellen geometrischen Mittelohrsituationen, wie sie beim Patienten in der Praxis oftmals vorgefunden werden können, als günstig erweisen kann.

Besonders günstig ist es, wenn die Stegelemente eine maximale Breite b und der radial äußere Ringbereich eine maximale Breite B aufweisen, wobei gilt: b < B, vorzugsweise 2b < B.

Um eine erwünschte Flexibilität zu erreichen, sollte bei einer maximalen Breite b der Stegelemente und einem minimalen Durchmesser D des plattenförmigen ersten Befestigungselements einschließlich des gegebenenfalls vorhandenen Ringbereiches gelten: b ≤ 0,05D, vorzugsweise b ≈ 0,03D. Beim Stand der Technik, etwa bei den in der US 2004/0162614 A1 beschriebenen Gehörknöchelchenprothesen, liegt das Verhältnis b/D mindestens bei 0,1 oder darüber.

Weiter ist es günstig, wenn das plattenförmige erste Befestigungselement der erfindungsgemäßen Gehörknöchelchenprothese eine Dicke, insbesondere eine Blechdicke t zwischen 0,01mm und 0,5mm, vorzugsweise zwischen 0,1mm und 0,25mm, sowie einen minimalen Durchmesser D zwischen 1,5mm und 8mm, vorzugsweise zwischen 2mm und 5mm, und die Stegelemente eine maximale Breite b zwischen 0,01mm und 0,3mm, vorzugsweise zwischen 0,05mm und 0,2mm aufweisen.

Um Verletzungen zu vermeiden sollte der Bruchbereich der abbrechbaren Stegelemente so gestaltet sein, dass nach Abbrechen als äußerste Kante kein Grat mit dem Trommelfell bzw. anderen anliegenden Strukturen direkten Kontakt erhält. Daher sind Ausführungsformen der erfindungsgemäßen Gehörknöchelchenprothese besonders zu bevorzugen, bei denen die Stegelemente jeweils in eine Einbuchtung des zentralen Ankoppelbereichs und/oder eines der radial äußeren Abschnitte münden, die den beim Abbrechen fast immer entstehenden Grat umgibt und diesen nach außen hin gewissermaßen abschirmt.

In der Praxis bewähren sich Weiterbildungen dieser Ausführungsformen, bei denen die Einbuchtung das entsprechende Stegelement in der Plattenebene des plattenförmigen ersten Befestigungselements jeweils in radialer Richtung so weit umschließt, dass das Stegelement auf mindestens 1/4, vorzugsweise auf mindestens 1/3 seiner Länge innerhalb der Einbuchtung verläuft.

Besonders vorteilhaft ist eine Weiterbildung dieser Ausführungsformen, die sich dadurch auszeichnet, dass die Sollbruchstelle in der Plattenebene des plattenförmigen ersten Befestigungselements innerhalb einer Einbuchtung angeordnet ist, in welcher dann die Bruchkante und der beim Abbrechen des Stegelements entstehende Grat verborgen ist.

Bevorzugt ist auch eine Klasse von Ausführungsformen der Erfindung, bei denen mindestens ein Verlängerungsteil vorgesehen ist, das wie ein Puzzle-Teil am Außenrand des plattenförmigen ersten Befestigungselements in der Plattenebene von der Seite her angeknöpft werden kann. Dadurch eröffnen sich hinsichtlich der geometrischen Form des Befestigungselements enorm viele Gestaltungsmöglichkeiten.

Insbesondere müssen jetzt nicht mehr unbedingt symmetrische Formen implantiert werden, wie sie bisher durch die vorhandenen Standard-Prothesen zwangsläufig vorgegeben waren. Vielmehr kann der Operateur ganz leicht und noch während der Operation eine handgefertigte, optimal angepasste Gehörknöchelchenprothese zielgenau "tailor-made" herstellen.

Oftmals liegt der Fall vor, dass der Hammergriff am Trommelfell vorhanden ist - oder eben auch fehlt - je nach dem wie oft voroperiert, welche Operationsmethode angewendet und welche spezifische Maßnahme ergriffen wurde. Entsprechend kann bei einer erfindungsgemäß flächenvariabel gestalteten Kopfplatte durch den Operateur zielgenau und exakt richtig reagiert werden, also der für den Hammergriff verantwortliche Bereich abgenommen oder - nach Bedarf - angefügt werden. Bei vorteilhaften Weiterbildungen dieser Klasse von Ausführungsformen ist dazu das seitlich anknöpfbare Verlängerungsteil als Appendix für den Hammer oder den Hammergriff der Gehörknöchelchenprothese geformt und ragt im zusammengeknöpften Zustand spitz vom Rand des plattenförmigen ersten Befestigungselements radial nach außen weg.

Diese Weiterbildungen lassen sich noch dadurch verbessern, dass das seitlich anknöpfbare Verlängerungsteil federnd im ersten Befestigungselement verankert ist, was insbesondere ein unbeabsichtigtes Abbrechen des sehr feinen Miniaturteils beim Anknöpfen in das Befestigungselement erschwert.

Die federnde Verankerung des Verlängerungsteils lässt sich beispielsweise mittels eines einfachen Klammerelements erreichen.

Bei weiteren vorteilhaften Varianten ist das seitlich anknöpfbare Verlängerungsteil einrastend und damit verliersicher im ersten Befestigungselement verankert, insbesondere mittels Widerhaken.

In der Regel wird bei der erfindungsgemäßen GehörknöchelchenProthese das Verbindungselement zwischen den Befestigungselementen als länglicher Schaft ausgeführt, wie dies an sich aus dem Stand der Technik wohlbekannt ist.

Um die oben erörterte Flexibilität bzw. Variabilität der Prothese - wie sie an sich in der EP 1 181 907 B1 beschrieben ist - zu erhöhen, kann bei einer besonders bevorzugten Weiterbildung dieser Ausführungsform am oder im länglichen Schaft mindestens ein Kugelgelenk vorgesehen sein. Vorteilhaft im Hinblick auf eine besonders hohe postoperative Beweglichkeit der Prothese sind Varianten, bei denen der längliche Schaft eine Vielzahl von aneinander angrenzenden weiteren Drehelementen, vorzugsweise eine Kugelgelenkkette, umfasst.

Alternativ kann der Schaft bei besonders einfachen und preisgünstig herstellbaren Ausführungsformen der erfindungsgemäßen Prothese aber auch einstückig durchgehend, insbesondere starr ausgeführt sein.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In vielen in der Praxis eingesetzten Ausführungsformen der Erfindung wird das erste Befestigungselement eine zur Anlage am Trommelfell ausgebildete Kopfplatte umfassen. Bei vielen weiteren Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke, als Stempel oder als Clip zur mechanischen Verbindung mit einem weiteren Glied der Gehörknöchelchenkette ausgebildet ist.

Bei Weiterbildungen dieser Ausführungsformen ist die Prothese über das als Kopfplatte ausgebildete erste Befestigungselement einerseits am Trommelfell und über das zweite Befestigungselement andererseits am Amboss oder am Steigbügel befestigt.

Alternative Ausgestaltungen können vorsehen, dass die Gehörknöchelchenprothese mittels Perforation der Steigbügelfußplatte (=Stapedektomie bzw. Stapedotomie) und/oder mittels Eröffnung der menschlichen Hörschnecke (=Cochleotomie) an dem der Trommelfellseite entgegen gesetzten anderen Ende direkt an das Innenohr angekoppelt ist, insbesondere über einen Kolben.

Möglich sind Ausführungsformen der Erfindung, bei denen die Prothese oder Teile davon aus biokompatiblen Kunststoffen, insbesondere Silikon, Polytetrafluorethylen (PTFE) oder Polyetheretherketon (PEEK), und/oder aus Faserverbundwerkstoffen, insbesondere Kohlefasern hergestellt sind. Mit diesen Materialien können postoperative Abstoßungsreaktionen in den meisten Fällen verhindert werden.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgebildetes Befestigungselement sollte bei der erfindungsgemäßen Gehörknöchelchenprothese grundsätzlich eine wachstumsfördernde Beschichtung, ein direkt ins Innenohr führendes, etwa in Form eines Kolbens ausgebildetes Befestigungselement hingegen eine wachstumshemmende Beschichtung aufweisen.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgegebenen oder vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels einer individuellen ausgestalteten Gehör-knöchelchenprothese erreichen.

Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Gehörknöchelchenprothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

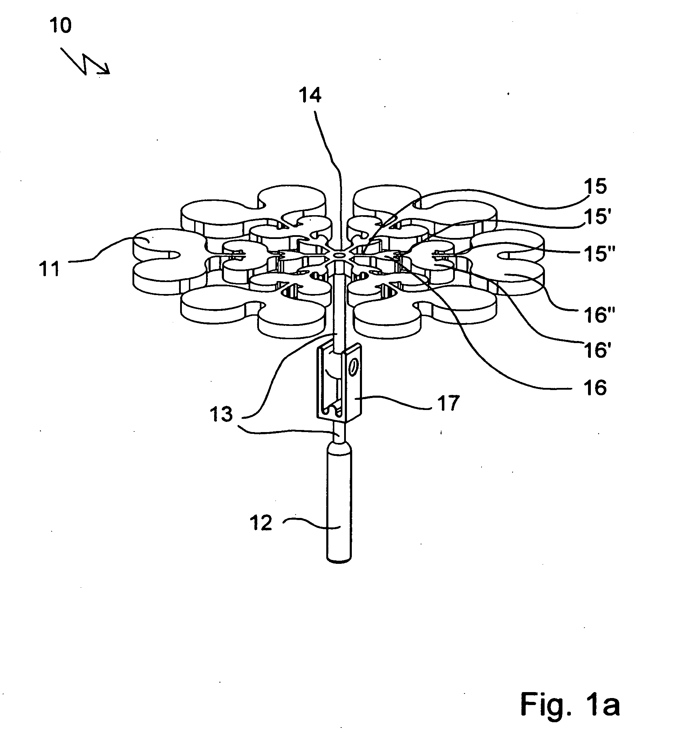

Die Figuren 1a-b, 2a-b und 3a-b der Zeichnung sind in 2er-Gruppen unterteilt, wobei die jeweiligen Einzelfiguren einer Gruppe voneinander durch eine Nummerierung mit a, b unterschieden sind. Die "a"-Figuren enthalten jeweils eine schematische räumliche Darstellung einer Ausführungsform der erfindungsgemäßen Gehörknöchelchenprothese, während die "b"-Figuren das zur entsprechenden "a"-Figur gehörige, erfindungsgemäß ausgestaltete erste Befestigungselement im Detail zeigen. Im Übrigen sind in der Zeichnung Elemente mit gleichem Aufbau und/oder gleicher Funktion mit der derselben Bezugsziffer gekennzeichnet.

### Im Einzelnen zeigen:

- Fign. 1a-b: eine Ausführungsform der erfindungsgemäßen Gehörknöchelchenprothese mit einem als Trommelfell-Kopfplatte gestalteten, sternförmig aufgebauten ersten Befestigungselement, einem Kugelgelenk im Verbindungselement und einem kolbenförmigen zweiten Befestigungselement;

- Fign. 2a-b: eine Ausführungsform mit zwei plattenförmig aufgebauten Befestigungselementen;
- Fign. 3a-b: eine Ausführungsform mit einem als Appendix für den Hammer oder den Hammergriff geformten Verlängerungsteil am ersten Befestigungselement sowie einem klammerförmigen zweiten Befestigungselement;
- Fig. 4: eine Ausführungsform mit einem ersten Befestigungselement wie in Fig. 3a sowie einer geschlitzten Glocke als zweitem Befestigungselement; und
- Fig. 5: eine schematische Darstellung eines von einer Einbuchtung umgebenen Stegelements mit Sollbruchstelle.

Die erfindungsgemäßen **Gehörknöchelchenprothesen 10; 20; 30; 40** weisen jeweils an einem Ende ein plattenförmiges **erstes Befestigungselement 11; 21; 31** auf, welches in Form einer Kopfplatte zur Anlage am Trommelfell oder als Fußplatte zur Anlage an der Steigbügelfußplatte ausgebildet ist. Am anderen Ende der Gehörknöchelchenprothesen 10; 20; 30; 40 sitzt jeweils ein **zweites Befestigungselement 12; 22; 32; 42** zur mechanischen Verbindung der Prothese mit einem Glied oder Teilen eines Gliedes der Gehörknöchelchen-Kette oder direkt mit dem Innenohr. Dazwischen ist ein die beiden Befestigungselemente Schall leitend miteinander verbindendes **Verbindungselement 13; 23; 33** angeordnet, welches bei den gezeigten Ausführungsformen in Form eines ein- oder mehrteiligen, kurzen oder längeren Schaftes ausgeführt ist.

Das plattenförmige erste Befestigungselement 11; 21; 31 weist jeweils einen radial inneren, insbesondere um seinen Flächenschwerpunkt zentral angeordneten **Ankoppelbereich 14; 24; 34; 54** zum mechanischen Ankoppeln des ersten Befestigungselements 11; 21; 31 an das Verbindungselement 13; 23; 33 sowie mehrere **Stegelemente 15,15',15"; 25,25'; 35,35',35"; 55** zur radialen Verbindung des radial inneren Ankoppelbereichs mit **radial äußeren Abschnitten 16,16',16"; 26,26'; 36,36',36"; 56** des ersten Befestigungselements 11; 21; 31 auf.

Die Stegelemente 15,15',15"; 25,25'; 35,35',35"; 55 sind geometrisch derart gestaltet und ihr Material so gewählt, dass die Stegelemente 15,15',15"; 25,25'; 35,35',35"; 55 leicht vom plattenförmigen ersten Befestigungselement 11; 21; 31 abgebrochen und die an ihnen anhängenden radial äußeren Abschnitte 16,16',16"; 26,26'; 36,36',36"; 56 vom ersten Befestigungselement 11; 21; 31 gelöst werden können, so dass der Außendurchmesser des ersten Befestigungselements 11; 21; 31 in diesem Bereich verkleinert wird.

Die in den Figuren 1a-b dargestellte Ausführungsform weist als erstes Befestigungselement 11 eine Kopfplatte und als Verbindungselement 13 einen zweigeteilten Schaft auf, der zwischen seinen beiden Teilen ein **Kugelgelenk 17** enthält, um die mechanische Flexibilität des Schaftes zu erhöhen. Das zweite Befestigungselement 12 an dem der Kopfplatte entgegen gesetzten Ende der in Fig. 1a dargestellten Gehörknöchelchenprothese 10 ist im vorliegenden Ausführungsbeispiel als Kolben zur direkten Ankopplung der Gehörknöchelchenprothese 10 an das Innenohr ausgebildet.

Das erste Befestigungselement 11 ist so aufgebaut, dass ausgehend vom zentralen Ankoppelbereich 14 sternförmig Stegelemente 15 radial nach außen abgehen, an denen jeweils ein radial äußerer Abschnitt 16 anhängt, und dass an jedem dieser radial äußeren Abschnitte 16 über weitere Stegelemente 15',15" jeweils weitere radial äußere Abschnitte 16',16" anhängen..

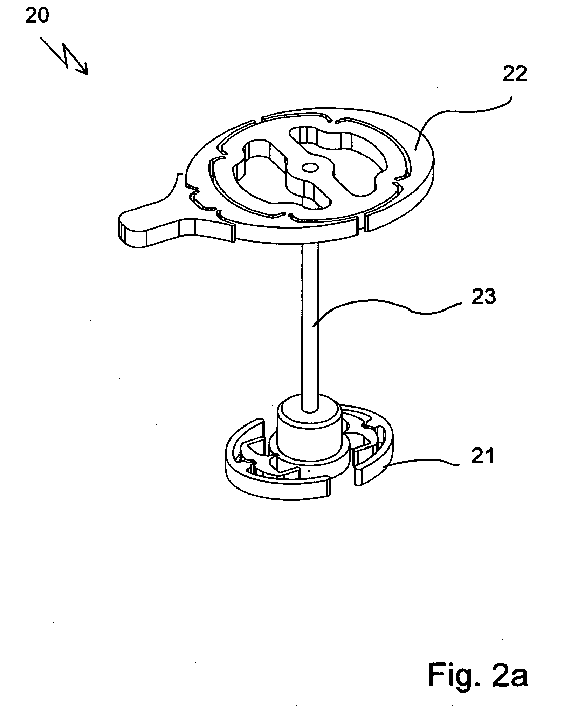

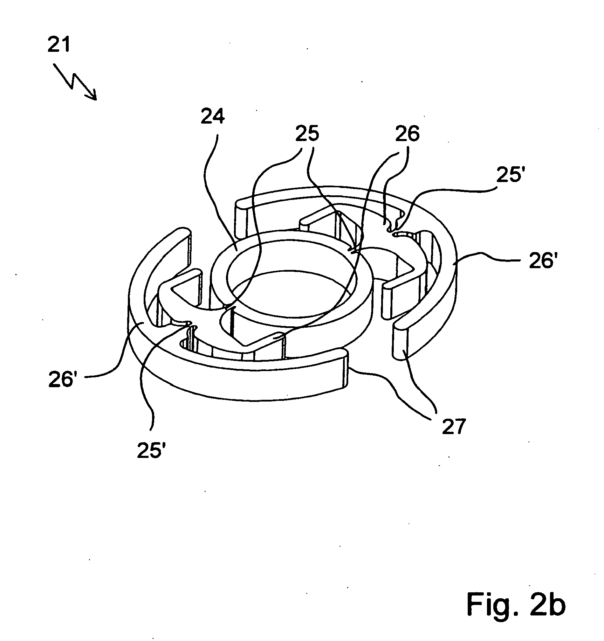

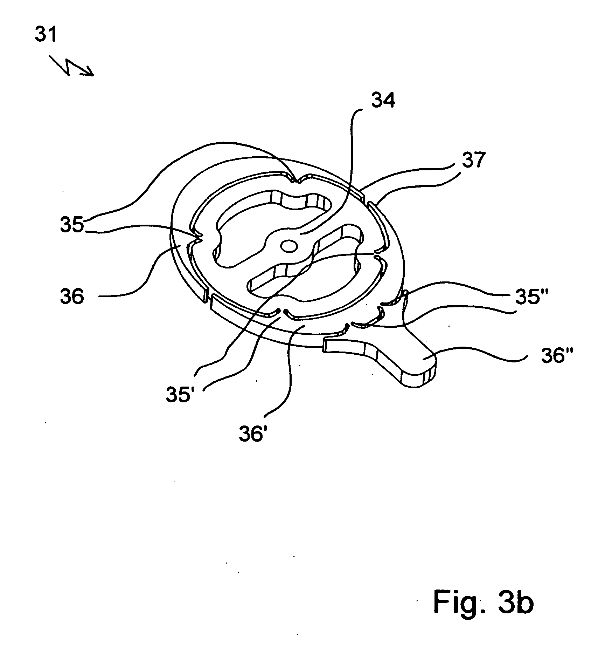

Bei der Ausführungsform nach den Figuren 2a-b sind alle beiden Befestigungselemente 21, 22 plattenförmig gestaltet und ihre Stegelemente 25, 25' erfindungsgemäß wiederum leicht abbrechbar, wobei das erste Befestigungselement 21 zur Anlage der Gehörknöchelchenprothese 20 auf der Steigbügelfußplatte ausgebildet ist, während das zweite Befestigungselement 22 als ebene Kopfplatte zur mechanischen Verbindung mit dem Trommelfell dient. Das zweite Befestigungselement 22 gleicht im Übrigen dem in Fig. 3b dargestellten ersten Befestigungselement 31.

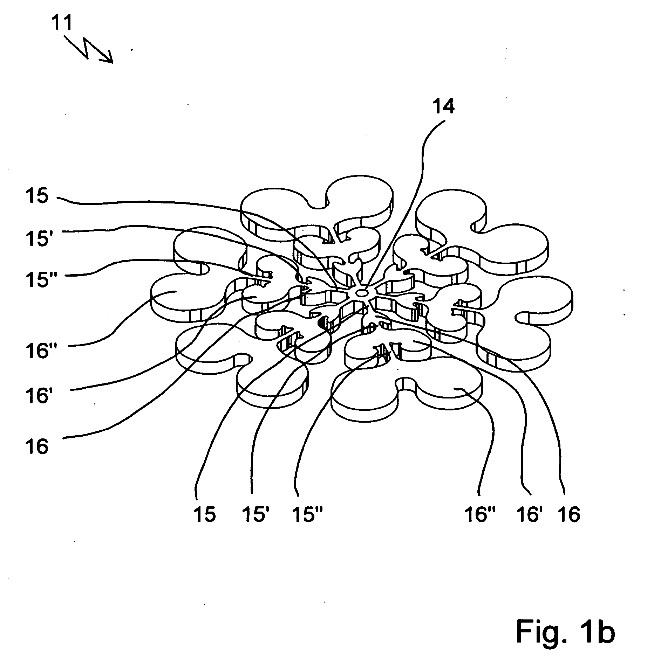

Da es sich hier um eine Totalprothese handelt, ist das Verbindungselement 23 als einstückig durchgehender, starrer langer Schaft ausgeführt. Auch beim ersten Befestigungselement 21 wird ein radial innerer Ankoppelbereich 24 über mehrere Stegelemente 25, 25' mit radial äußeren Abschnitten 26, 26' verbunden, die den Ankoppelbereich 24 umgeben, wobei auch hier der radial innere Ankoppelbereich 24 als Ringbereich gestaltet ist, allerdings mit einem größeren lichten Innendurchmesser als bei der Ausführungsform nach Fig. 1b. Wie in Fig. 2a gezeigt ist, kann daher der Ankoppelbereich 24 beispielsweise leicht auf eine kolbenförmige Verdickung des Verbindungselements 23 an seinem dem zweiten Befestigungselement 22 abgewandten Ende geklemmt werden.

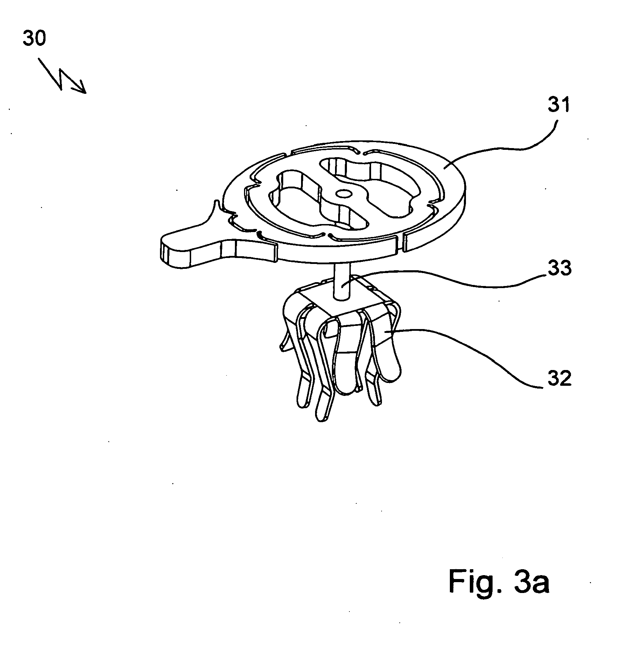

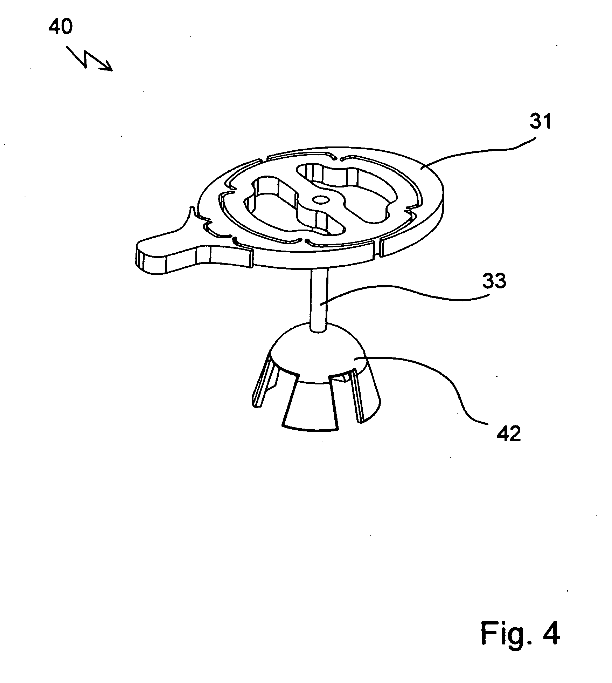

Sowohl bei dem in den Figuren 2a-b dargestellten Ausführungsbeispiel als auch bei den Ausführungsformen nach den Figuren 3a-b und 4 umgeben im ersten Befestigungselement 21; 31 jeweils radial äußere Abschnitte 26, 26' bzw. 36, 36' den radial inneren Ankoppelbereich 24; 34 in Form eines äußeren Ringbereiches 27; 37 mit Unterbrechungen in der Azimutalebene.

Die Ausführungsformen der Figuren 3a-b und 4 zeichnen sich weiterhin dadurch aus, dass das erste Befestigungselement 31 jeweils einen als Verlängerungsteil ausgestalteten Abschnitt 36" umfasst, der am Außenrand des plattenförmigen ersten Befestigungselements 31 in der Plattenebene seitlich von dem radial äußeren Abschnitt 36' spitz nach außen wegragt. Bei den in der Zeichnung dargestellten Ausführungsformen ist dieses seitliche Verlängerungsteil 36" als nasenförmiger Appendix für den Hammer oder den Hammergriff der Gehörknöchelchenprothesen 30; 40 geformt.

Das zweite Befestigungselement 32 bei der Gehörknöchelchenprothese 30 nach Fig. 3a ist als Klammer mit mehreren Zungen ausgeführt, während es bei der Gehörknöchelchenprothese 40 nach Fig. 4 die Form einer geschlitzten Glocke 42 aufweist. Beide Gestaltungen dienen zur Befestigung der jeweiligen Gehörknöchelchenprothese 30; 40 an einem Glied der Gehörknöchelchenkette, beispielsweise am Amboss oder am Steigbügel.

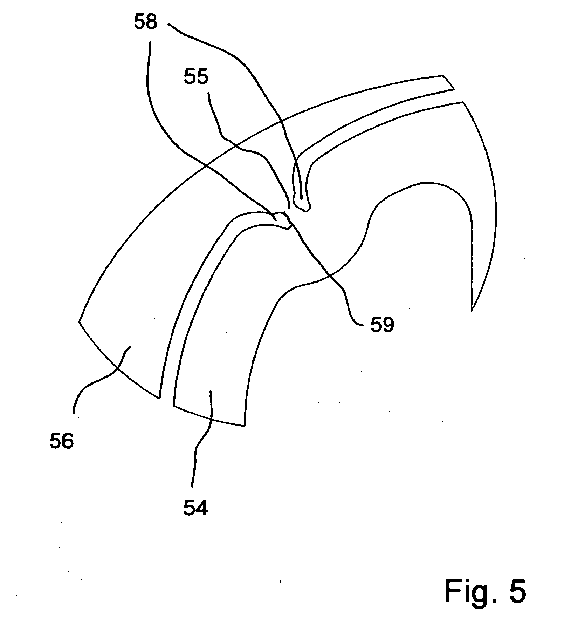

Wie in sämtlichen Figuren der Zeichnung angedeutet und in der schematischen Darstellung nach Fig. 5 im Detail gezeigt ist, münden die Stegelemente 15,15',15"; 25,25'; 35,35',35"; 55 jeweils in eine **Einbuchtung 58** des zentralen Ankoppelbereichs 14; 24; 34; 54 und/oder eines der radial äußeren Abschnitte 16,16',16"; 26,26'; 36,36',36"; 56. Die Einbuchtung 58 umschließt das entsprechende Stegelement 55 in der Plattenebene des plattenförmigen ersten Befestigungselements 11; 21; 31 jeweils in radialer Richtung so weit, dass das Stegelement 55 auf mindestens 1/4, vorzugsweise auf mindestens 1/3 seiner Länge innerhalb der Einbuchtung 58 verläuft.

Außerdem weisen dass die Stegelemente 15,15',15"; 25,25'; 35,35', 35"; 55 jeweils eine **Sollbruchstelle 59** mit minimaler Breite des jeweiligen Stegelements in diesem Bereich auf. Die Sollbruchstelle 59 ist in der Plattenebene des plattenförmigen ersten Befestigungselements 11; 21; 31 innerhalb der Einbuchtung 58 angeordnet.

Die Massenverteilung der einzelnen Teile der erfindungsgemäßen Gehörknöchelchenprothese 10; 20; 30; 40 kann in Abhängigkeit von einem gewünschten, vorgebbaren Frequenzgang der Schallleitung im Mittelohr berechnet sein, um ein individuelles Tuning der SchallleitungsEigenschaften zu ermöglichen.

Bei in der Zeichnung nicht eigens dargestellten weiteren Ausführungsformen der erfindungsgemäßen Gehörknöchelchenprothese können die zentralen Ankoppelbereiche und/oder die Stegelemente und/oder die radial äußeren Abschnitte auch andere Geometrien besitzen, um die gewünschte Flächenvariabilität des jeweiligen ersten Befestigungselements oder besonders gewünschte Festigkeitseigenschaften zu erzielen. So können etwa die radial äußeren Abschnitte einfach oder mehrfach unterbrochen, aber auch durchgehend ringförmig gestaltet sein, eine Schlangenlinienförmige Außenkontur und/oder Auskragungen oder Einbuchtungen in ihrer Azimutalebene, beispielsweise zur Aufnahme des Hammergriffs, aufweisen. Möglich sind auch Ausgestaltungen, bei denen Verlängerungsteile vorgesehen sind, die wie ein Puzzle-Teile am Außenrand des plattenförmigen ersten Befestigungselements in der Plattenebene von der Seite her angeknöpft werden können.

## Patentansprüche

1. Gehörknöchelchenprothese (10; 20; 30; 40), die mindestens ein Glied oder Teile eines Gliedes der Gehörknöchelchenkette ersetzt oder überbrückt, wobei die Gehörknöchelchenprothese (10; 20; 30; 40) an ihrem einen Ende ein im Wesentlichen plattenförmiges erstes Befestigungselement (11; 21; 31) zur Anlage am Trommelfell oder an der Steigbügelfußplatte und an ihrem anderen Ende ein zweites Befestigungselement (12; 22; 32; 42) zur mechanischen Verbindung mit einem Glied oder Teilen eines Gliedes der Gehörknöchelchenkette oder mit dem Innenohr sowie ein die beiden Befestigungselemente (11,12; 21,22; 31,32; 31,42) Schall leitend miteinander verbindendes Verbindungselement (13; 23; 33) umfasst, und wobei das plattenförmige erste Befestigungselement (11; 21; 31) einen radial inneren, insbesondere um den Flächenschwerpunkt des plattenförmigen ersten Befestigungselements (11; 21; 31) zentral angeordneten Ankoppelbereich (14; 24; 34; 54) zum mechanischen Ankoppeln des ersten Befestigungselements (11; 21; 31) an das Verbindungselement (13; 23; 33) sowie mehrere Stegelemente (15,15',15"; 25,25'; 35,35',35"; 55) zur radialen Verbindung des radial inneren Ankoppelbereichs (14; 24; 34; 54) mit radial äußeren Abschnitten (16,16',16"; 26,26'; 36,36',36"; 56) des ersten Befestigungselements (11; 21; 31) aufweist, **dadurch gekennzeichnet, dass** die Stegelemente (15,15',15"; 25,25'; 35,35',35"; 55) geometrisch derart gestaltet sind und ihr Material so gewählt ist, dass die Stegelemente (15,15',15"; 25,25'; 35,35',35"; 55) vom plattenförmigen ersten Befestigungselement (11; 21; 31) abgebrochen und die an ihnen anhängenden radial äußeren Abschnitte (16,16',16"; 26,26'; 36,36',36"; 56) vom ersten Befestigungselement (11; 21; 31) gelöst werden können, so dass der Außendurchmesser des ersten Befestigungselements (11; 21; 31) in diesem Bereich verkleinert wird,
wobei
die Stegelemente (15,15',15"; 25,25'; 35,35',35"; 55) jeweils eine Sollbruchstelle (59) mit minimaler Breite des jeweiligen Stegelements aufweisen.

2. Gehörknöchelchenprothese nach Anspruch 1, **dadurch gekennzeichnet, dass** beide Befestigungselemente (21, 22) plattenförmig mit abbrechbaren Stegelementen (25, 25') und daran anhängenden radial äußeren Abschnitten (26, 26') aufgebaut sind, wobei das erste Befestigungselement (21) zur Anlage der Gehörknöchelchenprothese (20) auf der Steigbügelfußplatte ausgebildet ist und das zweite Befestigungselement (22) als ebene Kopfplatte zur mechanischen Verbindung mit dem Trommelfell dient.

3. Gehörknöchelchenprothese nach einem der vorhergehenden Ansprüche, **dadurch gekennzeichnet, dass** das plattenförmige erste Befestigungselement (11; 21; 31) eine Dicke, insbesondere eine Blechdicke t zwischen 0,01mm und 0,5mm sowie einen minimalen Durchmesser D zwischen 1,5mm und 8mm und die Stegelemente (15,15',15"; 25,25'; 35,35',35"; 55) eine maximale Breite b zwischen 0,01mm und 0,2mm aufweisen.

4. Gehörknöchelchenprothese nach einem der Ansprüche 1 bis 3, **dadurch gekennzeichnet, dass** ausgehend vom zentralen Ankoppelbereich (14) sternförmig Stegelemente (15,15',15") radial nach außen abgehen, an denen jeweils ein radial äußerer Abschnitt (16) anhängt, und dass an jedem dieser radial äußeren Abschnitte (16) über weitere Stegelemente (15',15") jeweils weitere radial äußere Abschnitte (16',16") anhängen.

5. Gehörknöchelchenprothese nach einem der Ansprüche 1 bis 3, **dadurch gekennzeichnet, dass** die radial äußeren Abschnitte (26,26'; 36,36',36") des plattenförmigen ersten Befestigungselements (21; 31) einen äußeren Ringbereich (27; 37) bilden.

6. Gehörknöchelchenprothese nach Anspruch 5, **dadurch gekennzeichnet, dass** der radial äußere Ringbereich (27; 37) mindestens eine, vorzugsweise mehrere Unterbrechungen aufweist.

7. Gehörknöchelchenprothese nach einem der Ansprüche 5 oder 6, **dadurch gekennzeichnet, dass** der radial äußere Ringbereich (27; 37) eine ovale oder kreisrunde Form oder eine einseitige Einbuchtung zur Aufnahme des Hammergriffs oder in der Plattenebene des plattenförmigen ersten Befestigungselements (31) eine radial nach außen verlaufende Ausbuchtung (36") oder in der Plattenebene des plattenförmigen ersten Befestigungselements eine Schlangenlinienförmige Außenkontur aufweist.

8. Gehörknöchelchenprothese nach einem der Ansprüche 5 bis 7, **dadurch gekennzeichnet, dass** die Stegelemente (25,25'; 35,35', 35") eine maximale Breite b und der radial äußere Ringbereich (27; 37) eine maximale Breite B aufweisen, und dass gilt: b < B, vorzugsweise 2b < B.

9. Gehörknöchelchenprothese nach einem der vorhergehenden Ansprüche, **dadurch gekennzeichnet, dass** die Stegelemente (15,15',15"; 25,25'; 35,35',35") eine maximale Breite b und das plattenförmige erste Befestigungselement (11; 21; 31), gegebenenfalls einschließlich Ringbereich (27; 37), einen minimalen Durchmesser D aufweisen, und dass gilt: b ≤ 0,05D, vorzugsweise b ≈ 0,03D.

10. Gehörknöchelchenprothese nach einem der vorhergehenden Ansprüche, **dadurch gekennzeichnet, dass** die Stegelemente (15,15',15"; 25,25'; 35,35',35"; 55) jeweils in eine Einbuchtung (58) des zentralen Ankoppelbereichs (14; 24; 34; 54) und/oder eines der radial äußeren Abschnitte (16,16',16"; 26,26'; 36,36', 36"; 56) münden.

11. Gehörknöchelchenprothese nach Anspruch 10, **dadurch gekennzeichnet, dass** die Einbuchtung (58) das entsprechende Stegelement (15,15',15"; 25,25'; 35,35',35"; 55) in der Plattenebene des plattenförmigen ersten Befestigungselements (11; 21; 31) jeweils in radialer Richtung so weit umschließt, dass das Stegelement (15,15',15"; 25,25'; 35,35',35"; 55) auf mindestens 1/4, vorzugsweise auf mindestens 1/3 seiner Länge innerhalb der Einbuchtung (58) verläuft.

12. Gehörknöchelchenprothese nach einem der Ansprüche 10 oder 11, **dadurch gekennzeichnet, dass** die Sollbruchstelle (59) in der Plattenebene des plattenförmigen ersten Befestigungselements (11; 21; 31) innerhalb der Einbuchtung (58) angeordnet ist.

13. Gehörknöchelchenprothese nach einem der vorhergehenden Ansprüche, **dadurch gekennzeichnet, dass** das Verbindungselement (13; 23; 33) zwischen den Befestigungselementen (11, 12; 21,22; 31,32; 31,42) als länglicher Schaft ausgeführt ist und mindestens ein Gelenk, insbesondere ein Kugelgelenk (17), vorzugsweise eine Kugelgelenk-Kette aufweist.

14. Gehörknöchelchenprothese nach einem der Ansprüche 1 bis 13,
**dadurch gekennzeichnet, dass** das zweite Befestigungselement (22; 32; 42) als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

15. Gehörknöchelchenprothese nach einem der Ansprüche 1 bis 13, **dadurch gekennzeichnet, dass** die Gehörknöchelchenprothese (10) mittels Perforation der Steigbügelfußplatte (=Stapedektomie bzw. Stapedotomie) und/oder mittels Eröffnung der menschlichen Hörschnecke (=Cochleotomie) an dem der Trommelfellseite entgegen gesetzten anderen Ende direkt an das Innenohr ankoppelbar ist, insbesondere über einen Kolben (12).

## Claims

1. Ossicular prosthetic (10; 20; 30; 40), which replaces or bridges over at least one member or parts of a member of the ossicular chain, in which at one end the ossicular prosthetic (10; 20; 30; 40) comprises an essentially disc shaped first fastening element (11; 21; 31) for applying to the eardrum or stapes footplate and at the other end a second fastening element (12; 22; 32; 42) for mechanical connection to a member or parts of a member of the ossicular chain or the inner ear as well as a connecting element (13; 23; 33) connecting both fastening elements (11, 12; 21, 22; 31, 32; 31, 42) to each other in a way, which conducts sound, and in which the disc shaped first fastening element (11; 21; 31) has a radially inner coupling area (14; 24; 34; 54) arranged centrally, particularly around the centroid of the disc shaped first fastening element (11; 21; 31) for mechanical coupling of the first fastening element (11; 21; 31) to the connecting element (13; 23; 33) as well as several link elements (15, 15',15"; 25, 25'; 35, 35', 35"; 55) for radial connection of the radially inner coupling area (14; 24; 34; 54) to radially outer sections (16, 16', 16"; 26, 26'; 36, 36', 36"; 56) of the first fastening element (11; 21; 31), **characterised in that** the link elements (15, 15', 15"; 25, 25'; 35, 35', 35"; 55) are arranged geometrically in such a way and their material is selected so that the link elements (15, 15', 15"; 25, 25'; 35, 35', 35"; 55) may be broken off from the disc shaped first fastening element (11; 21; 31) and the radially outer sections (16, 16', 16"; 26, 26'; 36, 36', 36"; 56) attached to it may be removed from the first fastening element (11; 21; 31) so that the external diameter of the first fastening element (11; 21; 31) is reduced in this area, in which the link elements (15, 15', 15"; 25, 25'; 35, 35', 35"; 55) each have a predetermined breaking point (59) with a minimum width for each link element.

2. Ossicular prosthetic according to claim 1, **characterised in that** both fastening elements (21, 22) are made disc shaped with link elements (25, 25'), which may be broken off, and radially outer sections (26, 26') attached to them, in which the first fastening element (21) is made for applying the ossicular prosthetic (20) to the stapes footplate and the second fastening element (22) is used as a flat head plate for mechanical connection to the eardrum.

3. Ossicular prosthetic according to one of the previous claims, **characterised in that** the disc shaped first fastening element (11; 21; 31) has a thickness, particularly a sheet thickness t, of between 0.01 mm and 0.5 mm as well as a minimum diameter D of between 1.5 mm and 8 mm and the link elements (15, 15', 15"; 25, 25'; 35, 35', 35"; 55) have a maximum width b of between 0.01 mm and 0.2 mm.

4. Ossicular prosthetic according to one of claims 1 to 3, **characterised in that**, going out from the central coupling area (14), star shaped link elements (15, 15', 15") branch off radially outwards, to each of which a radially outer section (16) is attached, and that other radially outer sections (16', 16") are attached to each of these radially outer sections (16) through other link elements (15', 15").

5. Ossicular prosthetic according to one of claims 1 to 3, **characterised in that** the radially outer sections (26, 26'; 36, 36', 36") of the disc shaped first fastening element (21; 31) form an outer ring area (27; 37).

6. Ossicular prosthetic according to claim 5, **characterised in that** the radially outer ring area (27; 37) has at least one, preferably several, interruptions.

7. Ossicular prosthetic according to one of claims 5 or 6, **characterised in that** the radially outer ring area (27; 37) has an oval or circular shape or an indentation on one side for housing the hammer grip or a bulge (36") running radially outwards in the disc plane of the disc shaped first fastening element (31) or a wavy line shaped outer contour in the disc plane of the disc shaped first fastening element.

8. Ossicular prosthetic according to one of claims 5 to 7, **characterised in that** the link elements (25, 25'; 35, 35'; 35") have a maximum width b and the radially outer ring area (27; 37) has a maximum width B and that b < B, preferably 2 b < B, applies.

9. Ossicular prosthetic according to one of the previous claims, **characterised in that** the link elements (15, 15', 15"; 25, 25'; 35, 35', 35") have a maximum width b and the disc shaped first fastening element (11; 21; 31), if necessary including the ring area (27; 37), has a minimum diameter D, and that b ≤ 0.05 D, preferably b ≈ 0.03 D, applies.

10. Ossicular prosthetic according to one of the previous claims, **characterised in that** the link elements (15, 15', 15"; 25, 25'; 35, 35', 35"; 55) each open into an indentation (58) in the central coupling area (14; 24; 34; 54) and/or one of the radially outer sections (16, 16', 16"; 26, 26'; 36, 36', 36"; 56).

11. Ossicular prosthetic according to claim 10, **characterised in that** the indentation (58) encloses the relevant link element (15, 15', 15"; 25, 25'; 35, 35', 35"; 55) in the disc plane of the disc shaped first fastening element (11; 21; 31) each in a radial direction to the extent that the link element (15, 15', 15"; 25, 25'; 35, 35', 35"; 55) runs inside the indentation (58) for at least 1/4, preferably 1/3, of its length.

12. Ossicular prosthetic according to one of claims 10 or 11, **characterised in that** the predetermined breaking point (59) in the disc plane of the disc shaped first fastening element (11; 21; 31) is arranged inside the indentation (58).

13. Ossicular prosthetic according to one of the previous claims, **characterised in that** the connecting element (13; 23; 33) is made as a elongated shaft between the fastening elements (11, 12; 21, 22; 31, 32; 31, 42) and has at least one joint, particularly a ball and socket joint (17), preferably a ball and socket joint chain.

14. Ossicular prosthetic according to one of claims 1 to 13, **characterised in that** the second fastening element (22; 32; 42) is made as a disc, sleeve, loop, closed cap, cap with a single or several slits or as a clip for mechanical connection to a further member of the ossicular chain.

15. Ossicular prosthetic according to one of claims 1 to 13, **characterised in that** the ossicular prosthetic (10) may be coupled directly to the inner ear by means of perforation of the stapes footplate (= stapedectomy or stapedotomy) and/or by means of opening the human cochlea (= cochleotomy) at the end opposite the eardrum side, particularly through a pin (12).

## Revendications

1. Prothèse des osselets de l'oreille (10 ; 20 ; 30 ; 40), qui remplace ou joint au moins un élément ou des parties d'un élément de la chaîne d'osselets de l'oreille, dans laquelle la prothèse des osselets de l'oreille (10 ; 20 ; 30 ; 40) comprend, à une de ses extrémités, un premier élément de fixation (11 ; 21 ; 31) sensiblement en forme de plaque pour s'appuyer sur la membrane tympanique ou sur la plaque de base de l'étrier et, à son autre extrémité, un second élément de fixation (12 ; 22 ; 32 ; 42) pour se raccorder mécaniquement à un élément ou à des parties d'un élément de la chaîne d'osselets de l'oreille ou à l'oreille interne, ainsi qu'un élément de raccordement (13 ; 23 ; 33) reliant les deux éléments de fixation (11,12 ; 21,22 ; 31,32 ; 31,42) l'un à l'autre de manière à conduire le son, et dans laquelle le premier élément de fixation (11 ; 21 ; 31) en forme de plaque présente une zone de couplage radialement interne (14 ; 24 ; 34 ; 54) aménagée centralement en particulier autour du centre de gravité de surface du premier élément de fixation (11 ; 21 ; 31) en forme de plaque pour coupler mécaniquement le premier élément de fixation (11 ; 21 ; 31) sur l'élément de raccordement (13 ; 23 ; 33), ainsi que plusieurs traverses (15, 15', 15" ; 25,25' ; 35,35',35" ; 55) pour le raccordement radial de la zone de couplage radialement interne (14 ; 24 ; 34 ; 54) avec des sections radialement externes (16, 16', 16" ; 26, 26' ; 36,36', 36" ; 56') du premier élément de fixation (11 ; 21 ; 31), **caractérisée en ce que** l'on conforme géométriquement les traverses (15, 15', 15" ; 25, 25' ; 35, 35', 35" ; 55) et l'on choisit leur matériau de sorte que les traverses (15, 15', 15" ; 25, 25' ; 35, 35', 35" ; 55) puissent être brisées par le premier élément de fixation (11 ; 21 ; 31) en forme de plaque et que les sections radialement externes (16, 16', 16" ; 26, 26' ; 36, 36', 36" ; 56) s'accrochant sur celles-ci puissent être détachées par le premier élément de fixation (11 ; 21 ; 31) de manière à diminuer le diamètre externe du premier élément de fixation (11 ; 21 ; 31) dans cette zone,
et dans laquelle les traverses (15, 15', 15" ; 25, 25' ; 35, 35', 35" ; 55) présentent respectivement un point de rupture théorique (59) avec la largeur minimale de la traverse respective.

2. Prothèse des osselets de l'oreille selon la revendication 1, **caractérisée en ce que** les deux éléments de fixation (21, 22) sont constitués en forme de plaque avec des traverses cassables (25, 25') et des sections radialement externes (26, 26') qui s'y accrochent, dans laquelle le premier élément de fixation (21) est réalisé pour l'appui de la prothèse des osselets de l'oreille (20) sur la plaque de base de l'étrier et le second élément de fixation (22) sert de plaque de tête plane pour le raccordement mécanique avec la membrane tympanique.

3. Prothèse des osselets de l'oreille selon l'une quelconque des revendications précédentes, **caractérisée en ce que** le premier élément de fixation (11 ; 21 ; 31) en forme de plaque présente une épaisseur, en particulier une épaisseur de tôle t comprise entre 0,01 mm et 0,5 mm, ainsi qu'un diamètre minimal D compris entre 1,5 mm et 8 mm, tandis que les traverses (15, 15', 15" ; 25, 25' ; 35, 35', 35" ; 55) présentent une largeur maximale b comprise entre 0,01 mm et 0,2 mm.

4. Prothèse des osselets de l'oreille selon l'une quelconque des revendications 1 à 3, **caractérisée en ce que**, depuis la zone de couplage centrale (14), des traverses (15, 15', 15") en forme d'étoile, sur lesquelles s'accroche respectivement une section radialement externe (16), partent radialement vers l'extérieur et **en ce que** d'autres sections radialement externes (16', 16") s'accrochent respectivement sur chacune de ces sections radialement externes (16) par le biais d'autres traverses (15', 15").

5. Prothèse des osselets de l'oreille selon l'une quelconque des revendications 1 à 3, **caractérisée en ce que** les sections radialement externes (26, 26' ; 36, 36', 36") du premier élément de fixation (21 ; 31) en forme de plaque forment une zone annulaire externe (27 ; 37).

6. Prothèse des osselets de l'oreille selon la revendication 5, **caractérisée en ce que** la zone annulaire radialement externe (27 ; 37) présente au moins une interruption, de préférence plusieurs interruptions.

7. Prothèse des osselets de l'oreille selon l'une quelconque des revendications 5 ou 6, **caractérisée en ce que** la zone annulaire radialement externe (27 ; 37) présente une forme ovale ou circulaire ou un creux d'un côté pour recevoir le manche du marteau ou un renflement (36") s'étendant radialement vers l'extérieur dans le plan de plaque du premier élément de fixation (31) en forme de plaque ou un contour externe en forme sinueuse dans le plan de plaque du premier élément de fixation en forme de plaque.

8. Prothèse des osselets de l'oreille selon l'une quelconque des revendications 5 à 7, **caractérisée en ce que** les traverses (25, 25' ; 35, 35', 35") présentent une largeur maximale b, tandis que la zone annulaire radialement externe (27 ; 37) présente une largeur maximale B et b < B, de préférence 2b < B.

9. Prothèse des osselets de l'oreille selon l'une quelconque des revendications précédentes, **caractérisée en ce que** les traverses (15, 15', 15" ; 25, 25' ; 35, 35', 35") présentent une largeur maximale b, le premier élément de fixation (11 ; 21 ; 31) en forme de plaque, éventuellement y compris la zone annulaire (27 ; 37), présente un diamètre minimal D et b ≤ 0,05D, de préférence b ≈ 0,03D.

10. Prothèse des osselets de l'oreille selon l'une quelconque des revendications précédentes, **caractérisée en ce que** les traverses (15, 15', 15" ; 25, 25' ; 35, 35', 35" ; 55) débouchent respectivement dans un creux (58) de la zone de couplage centrale (14 ; 24 ; 34 ; 54) et/ou d'une des sections radialement externes (16, 16', 16" ; 26, 26' ; 36, 36', 36" ; 56).

11. Prothèse des osselets de l'oreille selon la revendication 10, **caractérisée en ce que** le creux (58) entoure la traverse correspondante (15, 15', 15" ; 25, 25' ; 35, 35', 35" ; 55) dans le plan de plaque du premier élément de fixation (11 ; 21 ; 31) en forme de plaque, respectivement dans la direction radiale, pour autant que la traverse (15, 15', 15" ; 25, 25' ; 35, 35', 35" ; 55) s'étende sur au moins 1/4, de préférence sur au moins 1/3 de sa longueur dans le creux (58).

12. Prothèse des osselets de l'oreille selon l'une quelconque des revendications 10 ou 11, **caractérisée en ce que** le point de rupture théorique (59) est aménagé dans le plan de plaque du premier élément de fixation (11 ; 21 ; 31) en forme de plaque à l'intérieur du creux (58).

13. Prothèse des osselets de l'oreille selon l'une quelconque des revendications précédentes, **caractérisée en ce que** l'élément de raccordement (13 ; 23 ; 33) se présente entre les éléments de fixation (11,12 ; 21,22 ; 31,32 ; 31,42) sous la forme d'une tige allongée et présente au moins une articulation, en particulier une articulation à rotule (17), de préférence une chaîne d'articulations à rotule.

14. Prothèse des osselets de l'oreille selon l'une quelconque des revendications 1 à 13, **caractérisée en ce que** le second élément de fixation (22 ; 32 ; 42) se présente sous la forme d'une plaque, d'une douille, d'une boucle, d'une cloche fermée, d'une cloche à une ou plusieurs fentes ou d'une agrafe pour le raccordement mécanique avec un autre élément de la chaîne d'osselets de l'oreille.

15. Prothèse des osselets de l'oreille selon l'une quelconque des revendications 1 à 13, **caractérisée en ce que** la prothèse des osselets de l'oreille (10) peut être couplée directement à l'oreille interne au moyen d'une perforation de la plaque de base de l'étrier (= stapédectomie ou stapédotomie) et/ou au moyen d'une incision de la cochlée humaine (= cochléotomie) à l'autre extrémité opposée au côté de la membrane tympanique, en particulier par le biais d'un piston (12).
